Europäisches Patentamt

European Patent Office

Office européen des brevets

(11) Veröffentlichungsnummer: **0 443 120 A1**

## EUROPÄISCHE PATENTANMELDUNG

(21) Anmeldenummer: 90123103.5

(22) Anmeldetag: 03.12.90

(51) Int. Cl.5: **A61B 5/04**

(30) Priorität: 22.01.90 CH 186/90

(43) Veröffentlichungstag der Anmeldung:
28.08.91 Patentblatt 91/35

(84) Benannte Vertragsstaaten:
AT BE CH DE FR GB IT LI

(71) Anmelder: **STUDER REVOX AG**
**Althardstrasse 30**
**CH-8105 Regensdorf ZH(CH)**

(72) Erfinder: **Schmid, Johann Jakob**
**Rosengartenstrasse 33**
**CH-8107 Buchs(CH)**

(74) Vertreter: **Ellenberger, Maurice**
**Willi Studer AG Althardstrasse 30**
**CH-8105 Regensdorf(CH)**

(54) Verfahren zur Ermittlung von Messwerten am menschlichen und tierischen Körper.

(57) Um bei solchen Verfahren, bei denen aus den Messwerten Vektoren (2, 3) und Vektorschleifen (1) ermittelt werden, die beispielsweise die Herztaetigkeit darstellen, Veraenderungen an den Schleifen, die durch Veraenderungen am Herzen bewirkt sind, besser zu erfassen, wird vorgeschlagen, aus Vektoren, die zu aufeinanderfolgenden Zeiten ermittelt werden, ein Vektorprodukt (4) zu ermitteln und den Verlauf solcher Vektorprodukte ueber eine Periode zu verfolgen.

Fig.1

EP 0 443 120 A1

# VERFAHREN ZUR ERMITTLUNG VON MESSWERTEN AM MENSCHLICHEN UND AM TIERISCHEN KOERPER

Die Erfindung betrifft ein Verfahren zur Ermittlung von Messwerten am menschlichen und am tierischen Koerper, ausgehend von elektrischen Potentialen, die so am Koerper abgegriffen werden, dass daraus ein Vektor abgeleitet werden kann, der ein elektrisches Feld darstellt, das vom Herzen erzeugt wird und das die Potentiale erzeugt.

Solche Verfahren sind beispielsweise aus dem Patent US-4,569,357 und der Patentanmeldung EP-0278215 bekannt. Das US-Patent zeigt wie ein Vektor aus Potentialen abgeleitet werden kann, die durch das elektrische Feld entstehen, das das Herz eines Menschen oder Tieres erzeugt. Die EP-Patentanmeldung zeigt, dass solche Vektoren zu beliebigen Zeiten ermittelt werden koennen und dass aus zwei solchen Vektoren, die zu verschiedenen Zeiten ermittelt werden, weitere Groessen ermittelt werden koennen. Zum Beispiel Differenzvektoren zwischen zwei Vektoren oder die Flaeche, die vom Vektor pro Zeiteinheit ueberstrichen wird. Dies soll der moeglichst guten Erfassung der Form einer Schleife dienen, die durch die Spitze des Vektors mit der Zeit durchlaufen wird.

Der Nachteil dieser Verfahren besteht darin, dass krankhafte Veraenderungen, die im Herzen stattfinden koennen, nicht notwendigerweise veraenderte Lage oder Betrag eines maximalen Vektors zur Folge haben. Solche krankhafte Veraenderungen fuehren auch nicht immer zu entsprechenden Veraenderungen der Form der Schleife oder vorhandene Veraenderungen an der Schleife kommen bei den bekannten Darstellungsweisen nicht immer gut genug zum Ausdruck. Durch dreidimensionale Darstellung der Schleife koennte dieser Nachteil zwar mit entsprechend grossem Aufwand vermindert werden, doch waeren zusaetzliche Angaben, wie zum Beispiel ueber die Geschwindigkeit, die der Vektor an jeder Stelle der Schleife hat, notwendig um die wichtigsten Veraenderungen im Verlaufe und in der Art wie die Schleife durchlaufen wird, festzustellen.

Die Erfindung, wie sie in den Anspruechen gekennzeichnet ist, loest die Aufgabe, ein Verfahren zur Ermittlung von Messwerten zu schaffen, das die obengenannten Nachteile vermeidet und es erlaubt mit kleinem Aufwand, den Verlauf der Schleife des Vektors und dessen Abweichungen zu erfassen.

Bei der Erfindung wird dies erreicht, indem der Vektor, dessen Spitze im Laufe der Zeit eine Schleife durchlaeuft, zu verschiedenen aufeinanderfolgenden Zeiten ermittelt wird und indem aus je zwei solcher Vektoren ein Vektorprodukt gebildet wird. Aufeinanderfolgend ermittelte Vektorprodukte werden graphisch oder numerisch so dargestellt, dass deren zeitliche Veraenderung erkennbar wird.

Die durch die Erfindung erreichten Vorteile sind insbesondere darin zu sehen, dass Veraenderungen des Verlaufes oder der Form von Schleifen, die in zeitlichem Abstand am gleichen Menschen oder Tier erfasst werden, besser und leichter erkannt werden koennen. Insbesondere werden auch Veraenderungen bei der Erzeugung der Schleifen erkannt. Beispielsweise kann dank dem erfindungsgemaessen Verfahren auch erkannt werden, ob bestimmte Bereiche der Schleife vom Vektor zu einer anderen Zeit schneller oder langsamer durchlaufen werden. Dadurch dass die Veraenderungen an den Schleifen leichter und sicherer erkennbar werden, lassen sich auch Ischaemien genauer lokalisieren. Zudem wird es besser moeglich, eindeutig zwischen Ischaemien und anderen Ursachen, die die Schleifen veraendern, zu unterscheiden. Eine bessere Differenzierung ist damit ermoeglicht. Weiter laesst sich das erfindungsgemaesse Verfahren leicht mit anderen, ergaenzenden Methoden in Diagnosesystemen einsetzen.

Im folgenden wird die Erfindung anhand von lediglich einen Ausfuehrungsweg darstellenden Zeichnungen naeher erlaeutert. Es zeigt:

| | |
|---|---|
| Figur 1 | Schleifen im Raum in perspektivischer Darstellung, |
| Figur 2 | die Darstellung eines elektrischen Potentials in Funktion der Zeit in einer Dimension, |
| Figur 3 | eine weitere Schleife gemaess Figur 1 aber in vergroessertem Massstab, |
| Figur 4 | ebenfalls eine weitere Schleife wie in Figur 3 in vergroessertem Massstab, |
| Figur 5 | eine Darstellung der Betraege von Vektorprodukten, |
| Figur 6 | eine Darstellung von Signalen mehrerer Herzschlaege und |
| Figur 7 | eine weitere spezielle Darstellung von Vektorprodukten. |

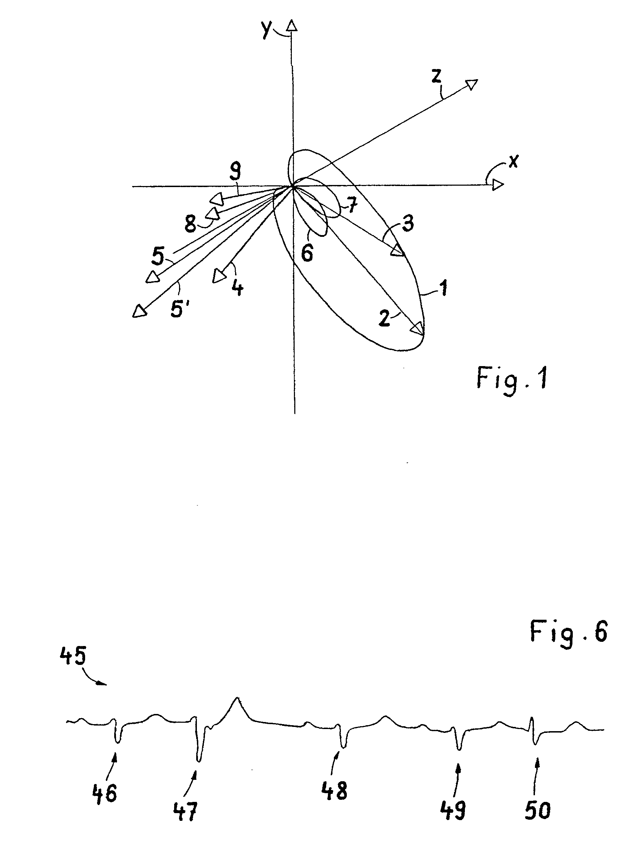

Figur 1 zeigt eine Schleife 1 als geometrischer Ort von Spitzen von Vektoren im Raum. Der Raum wird durch je eine x-, y- und z-Achse dargestellt, die sich beispielsweise auf einen menschlichen oder einen tierischen Koerper bezieht. Im Falle eines Menschen, ist die beispielsweise abgebildete Schleife 1 auch als QRS-Schleife bekannt. Sie ergibt sich aus der Position der Spitze eines Vektors zu verschiedenen Zeiten, der ein elektrisches Feld darstellt, das vom Herzen erzeugt wird. Dieser Vektor wird zu Zeiten ermittelt, die durch eine Folge von Zeiten, beispielsweise einer Abtastfolge, vorgegeben sind. Fuer die gezeigte Schleife 1 hat die Folge unendlich viele Zeiten, die zueinander einen unendlich kleinen Abstand haben. Bei abgetasteten Systemen hat aber die Folge eine endliche Anzahl Zeiten mit endlichem Abstand zueinander. Zu einer ersten Zeit ist ein Vektor 2 und zu einer zweiten Zeit ist ein Vektor 3 ermittelt und dargestellt. Ein Vektor 4 stellt dabei ein Vektorprodukt dar, das aus den beiden Vektoren 2 und 3 ermittelt ist. Der Vektor 4 steht senkrecht zu den beiden Vektoren 2 und 3. Er errechnet sich aus der Formel:

$V(t+T-t) = V(t) \times V(t+T)$ wobei

$V(t+T-t)$ dem Vektor 4, d.h. dem Vektorprodukt,

$V(t)$ dem Vektor 2 und

$V(t+T)$ dem Vektor 3 entspricht.

Die Vektoren 2 und 3 werden beispielsweise so ermittelt, wie es im Patent US 4,569,357 beschrieben ist. Bei vektorieller Addition der Vektorprodukte, stellt ein Vektor 5 die Summe der Vektorprodukte dar, die fuer die Schleife 1 errechnet werden. Die Vektorprodukte koennen aber auch numerisch addiert werden, wobei ein weiterer Vektor 5' entsteht. Eine ebenfalls gezeigte Schleife 6 ist auch als T-Schleife und eine weitere Schleife 7 als P-Schleife bekannt. Vektoren 8 und 9 sind Vektoren, die beispielsweise durch vektorielle Addition der Vektorprodukte der Schleifen 6 und 7 erhalten werden.

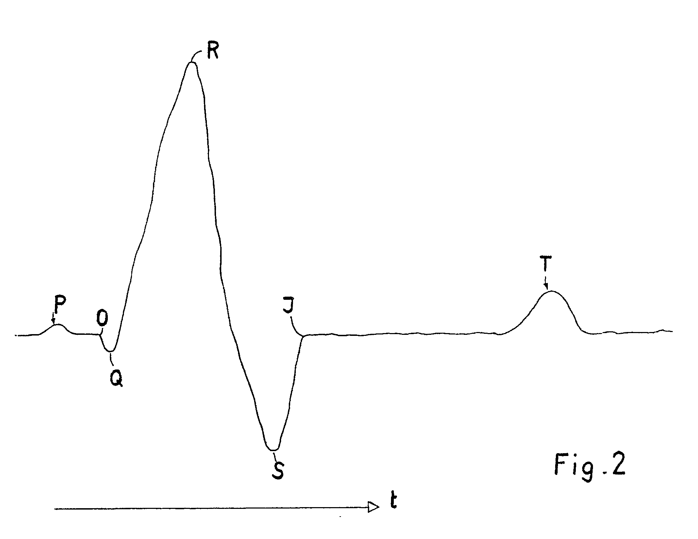

In Figur 2 sind elektrische Potentiale oder Potentialdifferenzen, beziehungsweise deren Werte, in Funktion der Zeit t aufgetragen. Dabei erkennt man die charakteristischen Punkte O, Q, R, S, J und die P- und T-Welle die aus der Elektrokardiographie des menschlichen Herzens bekannt sind. Durch die genannten Punkte koennen Anfang und Ende von verschiedenen Perioden definiert werden. Z.B. Repolarisation, Depolarisation, QRS-Komplex usw.

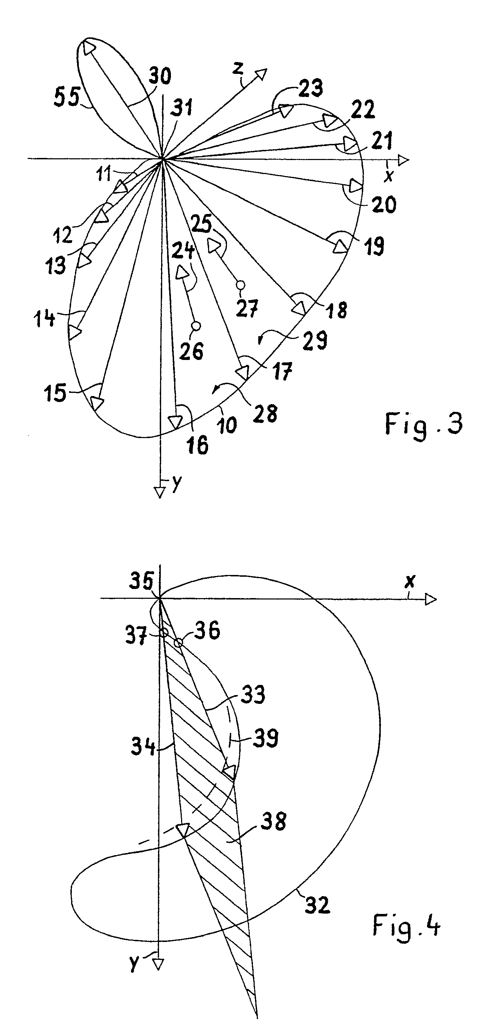

In Figur 3 ist eine weitere Schleife 10 im Raum dargestellt. Sie umfasst die Spitzen von Vektoren 11 bis 23 die zu Zeiten einer gegebenen Folge von Zeiten ermittelt sind. Aus jedem Paar von zwei Vektoren 11 und 12, 12 und 13 usw. lassen sich Vektorprodukte oder, was dasselbe bedeutet, Schwerpunktsvektoren ermitteln. Solche Schwerpunktsvektoren 24 und 25 sind in zwei Faellen eingezeichnet und greifen im Schwerpunkt 26 und 27 je eines Dreieckes 28 und 29 an, das durch die Vektoren 16, 17 und 17, 18 und nicht naeher dargestellter Differenzvektoren dazu gebildet wird. Eine Summe solcher Schwerpunktsvektoren bildet ein Summenvektor 30, der in einen Anfangs-und Endpunkt 31 der Schleife 10 nach den Regeln der Vektoranalysis verschoben ist. Der Vektor, der durch die Vektoren 11 bis 23 zu verschiedenen Zeiten dargestellt ist, bildet die Schleife 10 beispielsweise im Gegenuhrzeigersinn. Somit lassen sich auch laufend die Vektorprodukte aus den vorgaengig ermittelten Vektoren bilden. Damit kann man auch die Summe (numerisch oder vektoriell addiert) der Vektorprodukte oder Schwerpunktsvektoren laufend bilden. So erhaelt man den Summenvektor zu verschiedenen Zeiten, was durch eine Schleife 55 dargestellt ist, die die Spitze des Vektors so durchlaeuft.

In Figur 4 ist eine Schleife 32 mit einer speziellen Form im Raum gezeigt. Die spezielle Form besteht darin, dass Vektoren 33 und 34, die ja immer vom Anfangs-und Endpunkt 35 der Schleife 32 ausgehen, die Schleife 32 in Punkten 36 und 37 schneiden. Das Vektorprodukt der Vektoren 33 und 34 ergibt die Flaeche eines Parallelogrammes 38. Man sieht daraus, dass durch diese Vektorprodukte nicht die Flaeche der Schleife 32 berechnet wird. Trotzdem ist es sinnvoll diese Vektorprodukte zu ermitteln, denn sie geben auch in diesem speziellen Fall die gewuenschte Auskunft ueber den Verlauf der Schleife 32. Das erkennt man, wenn man untersucht, wie sich das Vektorprodukt aendert, wenn sich die Schleife 32 an dieser Stelle aendert und dort einen Verlauf aufweisen wuerde, wie ihn die Linie 39 zeigt. Die Schleife 32 kann infolge veraenderter Belastung des Herzens diesen anderen Verlauf aufweisen. In diesem Falle wuerden die Vektoren 33 und 34 kleiner ausfallen und das Vektorprodukt auch.

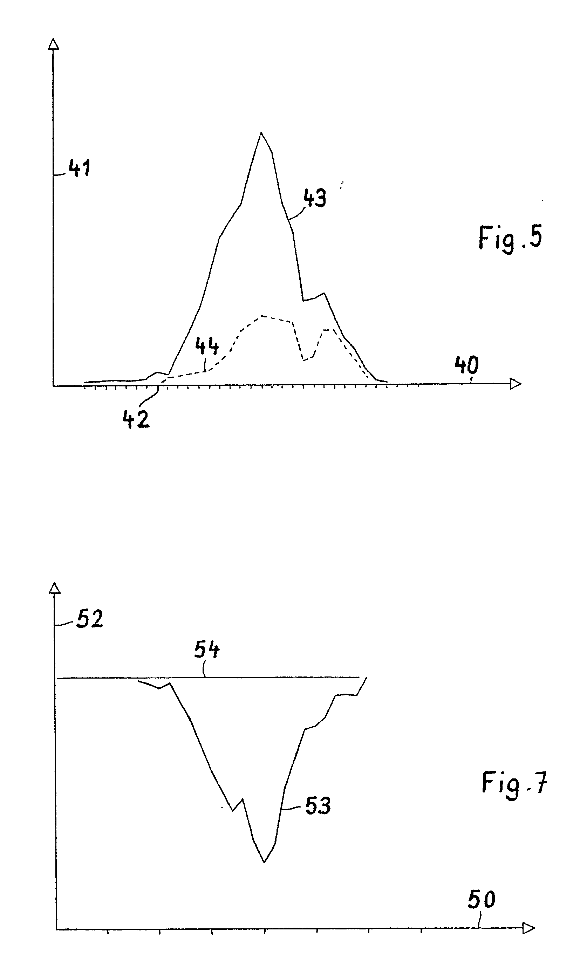

In Figur 5 sind ueber einer horizontalen Zeitachse 40 Werte von Vektorprodukten aufgetragen. Diese Werte lassen sich auf der vertikalen Achse 41 ablesen, wenn darauf eine Skala angebracht ist. Die Vektorprodukte sind zu Zeiten einer Folge ermittelt und entsprechende Markierungen 42 auf der Zeitachse 40 geben solche Zeiten an. Verbindet man die einzelnen Werte der Vektorprodukte, so erhaelt man eine Kurve 43, die die zeitliche Aenderung aufeinanderfolgender Vektorprodukte oder deren zeitlichen Ablauf zeigt. Die Kurve 43 entspricht Werten von Vektorprodukten, wie sie beispielsweise an einem Menschen mit unbelastetem Herzen ermittelt wurde. Eine weitere Kurve 44 zeigt Werte von Vektorprodukte wie sie am gleichen Menschen mit belastetem Herzen ermittelt wurde.

In Figur 6 ist ein Signal 45 der gleichen Art wie in Figur 2 aber fuer mehrere aufeinanderfolgende Herzschlaege dargestellt. Dieses Signal 45 besteht unter anderem aus fuenf QRS-Komplexen 46, 47, 48, 49 und 50. Diese QRS-Komplexe haben nicht alle vergleichbare Formen. Sie lassen sich drei verschiedenen Klassen zuordnen. Die Komplexe 46, 48 und 49 gehoeren zu einer ersten Klasse, der Komplex 47 gehoert zu einer zweiten Klasse und der Komplex 50 gehoert zu einer dritten Klasse. Die Komplexe 46, 48, 49 der ersten Klasse weisen demnach nur geringe Ausschlaege auf und haben insbesondere fast keinen Ausschlag nach oben. Der Komplex 47 der zweiten Klasse hat die staerksten Ausschlaege und der Komplex 50 der dritten Klasse hat nur kleine Ausschlaege aber sie sind nach oben und unten etwa gleich stark. Klassen koennen damit auf diese Weise unter Verwendung entsprechender vorgegebener Schwellwerte fuer die Ausschlaege definiert werden.

In Figur 7 sind ueber Achsen 51 und 52, die den Achsen 40 und 41 der Figur 5 entsprechen, Differenzwerte zwischen Werten dargestellt, die durch die Kurven 43 und 44 dargestellt werden. Diese Differenzwerte ergeben zusammen eine Kurve 53, die somit die Differenz zwischen vergleichbaren Werten von Vektorprodukten am unbelasteten und am belasteten Herzen zeigen. Diese Werte sind hier mit negativem Vorzeichen versehen und von einer Nulllinie 54 aus abgetragen. Sie ergeben sich demnach aus den Werten der Kurve 44, von denen man Werte der Kurve 43 zu entsprechenden Zeiten abgezaehlt hat.

In der Praxis wird das erfindungsgemaess Verfahren etwa folgendermassen durchgefuehrt:
Am Menschen oder am Tier, von dem die Messwerte gewonnen werden sollen, werden Elektroden angebracht und zwar beispielsweise so, wie dies im Patent US 4,569,357 beschrieben ist. Damit werden Potentialdifferenzen, wie sie die Figuren 2 und 6 zeigen, laufend erfasst und einem Rechner zugefuehrt, der hier nicht naeher beschrieben ist, der aber einem Geraet entsprechen kann, wie es im genannten US-Patent beschrieben ist. Der Rechner enthaelt beispielsweise einen an sich bekannten Taktgenerator, mit dem nun eine Taktfrequenz eingestellt, d.h. eine Folge von Zeiten vorgegeben wird. Damit werden die Potentiale zu diesen vorgegebenen Zeiten vom Rechner gemessen (quantifiziert) und fuer die weitere Verarbeitung periodisch zur Verfuegung gestellt. Ueber eine groessere Periode so erfasste Messwerte werden vorzugsweise einem Speicher zugefuehrt, damit sie von dort aus fuer weitere verschiedene Verarbeitungsschritte zur Verfuegung stehen. Aus diesen Messwerten werden jetzt die QRS-Komplexe, die T-Komplexe und die P-Komplexe ermittelt. Dazu muessen deren Anfangs-und Endpunkte gefunden werden. Diese werden vorzugsweise nach einem Verfahren ermittelt, das im Patent US 4,700,712 oder US 4,587,976 beschrieben ist. Damit ist das Signal oder sind die Messwerte in mehrere Perioden eingeteilt, deren Messwerte nun fuer sich untereinander verglichen werden koennen. Es koennen auch Grenz-oder Schwellwerte vorgegeben werden, die diese Messwerte klassieren. So koennen beispielsweise alle QRS-Komplexe nach Kriterien klassiert werden, wie sie fuer Figur 6 beschrieben sind. Aus so klassierten Messwerten koennen nun alle Komplexe der gleichen Klasse so gemittelt werden, dass fuer diese Klasse ein gemittelter Komplex entsteht. Beispielsweise koennte der QRS-Komplex aus Figur 2 ein solcher gemittelter durchschnittlicher QRS-Komplex sein. Dies kann auch fuer die Schleifen 1 (Fig. 1) 10 (Fig. 3) und 32 (Fig. 4) gelten, die ja solche QRS-Komplexe lediglich anders darstellen.

Wie ebenfalls aus dem genannten US-Patent 4,569,357 bekannt, werden aus den verschiedenen Werten, die zu jeder Zeit der Folge gemessen werden, nun auch die Vektoren berechnet. Daraus werden nach der oben angegebenen Formel auch die Vektorprodukte ermittelt. Daraus wiederum lassen sich entweder durch bekannte vektorielle Addition oder einfach durch Addition der Absolutwerte die Summe der Vektorprodukte ueber ganze Perioden berechnen, fuer die mehrere Vektorprodukte ermittelt wurden. Solche Perioden sind beispielsweise die gemittelten QRS-Komplexe, T-Komplexe und P-Komplexe. Dies kann auch fuer die Perioden der Depolarisation und der Repolarisation geschehen. Die Depolarisationsperiode beginnt mit dem O-Punkt vor einem QRS-Komplex und endet am sogenannten J-Punkt nach dem QRS-Komplex. Die Repolarisationsperiode beginnt am J-Punkt und endet am Ende des T-Komplexes. Alle diese Punkte sind aber schon dann bestimmt, wenn, wie oben angegeben, Anfang- und Ende der P-und T-Komplexe und des QRS-Komplexes gefunden sind. Anschliessende graphische Darstellung der in diesen Perioden ermittelten Schwerpunktsvektoren oder Vektorprodukten ueber einer Zeitachse gemaess Figur 5 und Figur 7 erlaubt es dann, Vergleiche zwischen solchen Messungen an verschiedenen Menschen oder am gleichen Menschen nach verschiedenen Zeiten oder bei verschiedenen Belastungen anzustellen. Entsprechende Erkenntnisse lassen sich gewinnen, wenn man die Schleife 55 der Vektorprodukte 30 zu verschiedenen Zeiten und bei verschiedenen Belastungen erfasst und vergleicht.

**Patentansprüche**

1. Verfahren zur Ermittlung von Messwerten am menschlichen und am tierischen Koerper, ausgehend von elektrischen Potentialen, die so am Koerper abgegriffen werden, dass daraus ein Vektor abgeleitet werden kann, der ein elektrisches Feld darstellt, das vom Herzen erzeugt wird und das die Potentiale erzeugt, dadurch gekennzeichnet, dass
eine Folge von Zeiten (42) vorgegeben wird, zu denen die Potentiale gemessen werden, dass
aus den Potentialen die zu einer ersten und einer zweiten Zeit gemessen werden, je ein erster und ein zweiter Vektor (2, 3) abgeleitet wird und dass
aus dem ersten und zweiten Vektor ein Vektorprodukt (4) ermittelt wird.

2. Verfahren nach Anspruch 1, dadurch gekennzeichnet, dass fuer alle Zeiten der Folge ein Vektor (11 bis 23) ermittelt wird und dass aus den so ermittelten Vektoren Anfangspunkte (31, O) und Endpunkte (31, J) von Perioden ermittelt werden, die die Taetigkeit des Herzens kennzeichnen.

3. Verfahren nach Anspruch 2, dadurch gekennzeichnet, dass das Vektorprodukt zu Zeiten der Folge ermittelt wird, die zu einer bestimmten Periode gehoeren und dass die innerhalb der Periode ermittelten Vektorprodukte addiert werden.

4. Verfahren nach Anspruch 1, dadurch gekennzeichnet, dass zu aufeinanderfolgenden Zeiten ermittelte Vektorprodukte so zueinander in Beziehung (43) gebracht werden, dass deren zeitliche Aenderung erkennbar wird.

5. Verfahren nach Anspruch 1, dadurch gekennzeichnet, dass fuer alle Zeiten der Folge ein Vektor und ein Vektorprodukt ermittelt werden und dass der zeitliche Verlauf der Vektorprodukte ermittelt wird.

6. Verfahren nach Anspruch 1, dadurch gekennzeichnet, dass fuer alle Zeiten der Folge ein Vektor und ein Vektorprodukt ermittelt werden und dass der raeumliche Verlauf (55) der Vektorprodukte ermittelt wird.

7. Verfahren nach Anspruch 5 oder 6, dadurch gekennzeichnet, dass der Verlauf der Vektorprodukte in einer Periode unter verschiedenen Bedingungen ermittelt wird.

8. Verfahren nach Anspruch 7, dadurch gekennzeichnet, dass der Verlauf fuer die verschiedenen Bedingungen nebeneinander graphisch dargestellt wird.

Fig.1

Fig.6

Fig.2

Fig.3

Fig.4

8

Fig.5

Fig.7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | US-A-4 700 712  (SCHMID)<br>* Figuren * * Spalte 3, Zeile 35 - Spalte 11, Zeile 41 *<br>- - - | 1,2 | A 61 B 5/04 |
| D,A | EP-A-0 278 215  (WILLI STUDER AG)<br>* Figuren * * Seite 2, Zeile 37 - Seite 5, Zeile 46 *<br>- - - - - | 1,2 | |

|  | RECHERCHIERTE SACHGEBIETE (Int. Cl.5) |
|---|---|
|  | A 61 B |

Der vorliegende Recherchenbericht wurde für alle Patentansprüche erstellt

| Recherchenort | Abschlußdatum der Recherche | Prüfer |
|---|---|---|
| Den Haag | 02 Mai 91 | CHEN A.H. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument